# EUROPÄISCHE PATENTANMELDUNG

(11) **EP 3 343 474 A1**
(43) Veröffentlichungstag der Anmeldung: **04.07.2018**
(21) Anmeldenummer: 16207352.2
(22) Anmeldetag: 29.12.2016
(51) Int. Cl.: G06Q 10/06

(54) **VERFAHREN ZUR AUSNUTZUNG DER KAPAZITÄT VON EINRICHTUNGEN IN EINEM SKIGEBIET, EINER MESSE, EINEM FREIZEITPARK ODER IN EINEM STADION**

(71) Anmelder: SKIDATA AG, 5083 Grödig/Salzburg (AT)
(72) Erfinder: Dr. HAIDACHER, Martin, 5020 Salzburg (AT); Dr. SCHLECHTER, Thomas, 5201 Seekirchen am Wallersee (AT)
(74) Vertreter: Karakatsanis, Georgios

(57) **Zusammenfassung**

Es wird ein Verfahren zur Ausnutzung der Kapazität von Einrichtungen in einem Skigebiet, einer Messe, einem Freizeitpark oder in einem Stadion vorgeschlagen, im Rahmen dessen die aktuelle Position und das Bewegungsprofil sämtlicher im Bereich des Skigebiets, der Messe, des Freizeitparks oder des Stadions anwesenden Personen erfasst werden, wobei auf einem Server eine People-Map erstellt wird, welche der räumlichen Verteilung der Personen entspricht, wobei durch die räumliche Verteilung der Personen als Funktion der Zeit ein Bewegungsprofil der Personen erstellt wird, wobei in Abhängigkeit der aktuellen Position der anwesenden Personen, des Bewegungsprofils der anwesenden Personen und der aktuellen Auslastung der Einrichtungen, alle oder einzelne Personen vom Server generierte Informationen erhalten, welche eine Redirektion dieser Personen bewirken können, wodurch die Ausnutzung der Kapazität der Einrichtungen optimiert werden kann.

## Beschreibung

Die vorliegende Erfindung bezieht sich auf ein Verfahren zur Ausnutzung der Kapazität von Einrichtungen in einem Skigebiet, einer Messe, einem Freizeitpark oder in einem Stadion gemäß dem Oberbegriff des Patentanspruchs 1.

Bei Skigebieten, Messen, Freizeitparks oder Stadien ist in der Regel eine Vielzahl von Einrichtungen vorgesehen; beispielsweise umfasst ein Skigebiet mehrere Pisten mit entsprechenden Fördereinrichtungen in Form von Liften, Restaurants, Hütten etc.

Hierbei tritt oft die Situation auf, dass bei einigen Einrichtungen Warteschlangen oder größeren Ansammlungen von Personen entstehen, wodurch zum einen der Komfort für die Personen beeinträchtigt wird und zum anderen die Kapazitäten der weiteren Einrichtungen nicht ausgenutzt werden; beispielsweise können in einem Skigebiet Warteschlangen vor Restaurants entstehen, während die Kapazität von Skiliften nicht ausgenutzt wird.

Der vorliegenden Erfindung liegt die Aufgabe zugrunde, ein Verfahren zur Ausnutzung der Kapazität von Einrichtungen in einem Skigebiet, einer Messe oder in einem Freizeitpark anzugeben.

Diese Aufgabe wird durch die Merkmale des Patentanspruchs 1 gelöst.

Demnach wird ein Verfahren zur Ausnutzung der Kapazität von Einrichtungen in einem Skigebiet, einer Messe, einem Freizeitpark oder in einem Stadion vorgeschlagen, im Rahmen dessen die aktuelle Position und das Bewegungsprofil sämtlicher im Bereich des Skigebiets, der Messe, des Freizeitparks oder des Stadions anwesenden Personen erfasst werden, wobei in einem Server eine People-Map erstellt wird, welche der räumlichen Verteilung der Personen entspricht, wobei durch die räumliche Verteilung der Personen als Funktion der Zeit ein Bewegungsprofil der Personen erstellt wird und wobei in Abhängigkeit der aktuellen Position der anwesenden Personen, des Bewegungsprofils und der aktuellen Auslastung der Einrichtungen, alle oder einzelne Personen von einem Server generierte Informationen erhalten, welche eine Redirektion dieser Personen bewirken können, wodurch die Ausnutzung der Kapazität der Einrichtungen optimiert werden kann.

Die Erfassung der Personen kann gemäß einer ersten Ausgestaltung der Erfindung mittels mobiler elektronischer Geräte, z.B. Mobiltelefone, welche die Personen bei sich tragen, über einen Standard zur Kommunikation in Funknetzwerken erfolgen. Beispielsweise können die Mobiltelefone der Personen über einen Funkstandard, z.B. über den Bluetooth Low Energy-Standard (BLE) oder über einen WLAN-Standard im Rahmen eines "Broadcasting" in regelmäßigen Abständen eine eindeutige ID senden, welche der IMEI - Nummer des Gerätes entsprechen kann, welche von mit einem Server verbundenen Sende-Empfangseinheiten, z.B. BLE Sende-Empfangseinheiten bzw. WLAN Sende-Empfangseinheiten in Reichweite des mobilen elektronischen Gerätes empfangen wird. Als Funkstandard kann beispielsweise auch der UWB-Standard (Ultra-Wide - Band) zur Nahbereichskommunikation verwendet werden, welcher eine Lokalisierung der mobilen elektronischen Geräte mit hoher Genauigkeit ermöglicht.

Hierbei erfolgt die Lokalisierung der mobilen elektronischen Geräte dadurch, dass anhand einer Referenzsignalstärke für den verwendeten Funkstandard, die in der Regel der Signalstärke in einem Meter Entfernung entspricht und die als Information im Signal des mobilen elektronischen Geräts enthalten ist oder in Abhängigkeit vom mobilen elektronischen Gerät, z.B. vom Typ des Mobiltelefons, in einer im Computer gespeicherten Tabelle abgelegt ist, der Abstand zwischen dem mobilen elektronischen Gerät und den sich in Reichweite befindenden Sende-Empfangseinheiten für den jeweiligen Funkstandard ermittelt wird, wobei anschließend eine Trilateration bzw. bei mehr als drei Sende-Empfangseinheiten eine Multilateration durchgeführt wird.

Alternativ kann die Lokalisierung der mobilen elektronischen Geräte dadurch erfolgen, dass diese die mittels eines in die mobilen elektronischen Geräte integrierten GPS-Moduls gewonnenen aktuellen GPS-Daten zusammen mit der eindeutigen ID über den verwendeten Funkstandard an die sich in Reichweite befindenden Sende-Empfangseinheiten senden.

Im Rahmen einer Weiterbildung wird vorgeschlagen, als mobiles elektronisches Gerät, mittels dessen die aktuelle Position sämtlicher im Bereich des Skigebiets, der Messe oder des Freizeitparks anwesenden Personen erfasst wird, eine portable Vorrichtung zu verwenden, die jeder Person beim Eintreten ausgehändigt wird, wenn diese nicht über diese Vorrichtung verfügen. Diese Vorrichtung umfasst ein Bauteil, welches die Kommunikation mit Sende-Empfangseinheiten des Skigebiets, der Messe oder des Freizeitparks mittels eines Funkstandards ermöglicht und optional eine Anzeigevorrichtung und ein Bauteil zur Ausgabe von akustischen Signalen. Die portable Vorrichtung ist jeweils einer Person zugeordnet.

Ferner kann die Vorrichtung ein GPS-Modul aufweisen, mittels dessen zum einen die genaue Position der Vorrichtung und zum anderen die Bewegungsrichtung und Geschwindigkeit, mit der sich die Vorrichtung bewegt, erfasst werden. Für den Fall eines Skigebietes ist die Vorrichtung derart ausgeführt, dass sie an einem der Skier oder an einem Snowboard derart befestigbar ist, dass die Anzeigevorrichtung beim Skifahren durch den Benutzer sichtbar ist; hierbei können durch die Anzeigevorrichtung, wenn keine vom Server generierte Information angezeigt wird, die Geschwindigkeit, die Höhe, die gefahrenen Höhenmeter etc. angezeigt werden. Ferner kann die Vorrichtung mit einem mobilen elektronischen Gerät des Benutzers über den Funkstandard gekoppelt werden, wodurch der Benutzer informiert werden kann, wenn die z.B. vor einem Restaurant abgestellten Skier bewegt werden, wodurch ein einfacher Diebstahlschutz realisiert wird.

Im Rahmen einer weiteren Ausgestaltung, die insbesondere für Freizeitparks geeignet ist, kann die portable Vorrichtung als AR/VR-Brille (Augmented Reality/Virtual Reality-Brille) ausgeführt sein.

Gemäß einer weiteren Ausgestaltung der Erfindung können die Personen anhand einer Personenerkennung mittels 3D-Kameras, beispielsweise TOF-Kameras, nämlich Kameras, welche mittels eines Laufzeitverfahrens (time of flight, TOF) Distanzen messen, Stereokameras oder PMD-Kameras, nämlich TOF-Kameras umfassend einen Photomischdetektor erfasst und lokalisiert werden, welche mittels Bildanalyse eine genaue Positionserfassung der Personen ermöglichen. Die Kameras sind mit dem Server verbunden. Die Erfassung mittels Kameras kann auch zusätzlich zur Erfassung mittels der mobilen elektronischen Geräte durchgeführt werden, um auf diese Weise die Lokalisierungsgenauigkeit zu erhöhen. Eine mittels eines mobilen elektronischen Gerätes am Eingang eines Skigebietes, einer Messe oder eines Freizeitpark erfasste und lokalisierte Person kann demnach zusätzlich mittels einer Kamera erfasst werden, wodurch der Person personenspezifische Informationen, wie z.B. Alter und Geschlecht zugeordnet werden können.

Ferner können Trinocularkameras verwendet werden, welche jeweils drei Einzel-Kameras unterschiedlichen Abstands zueinander umfassen, die in einer Reihe angeordnet sind, wobei jeweils zwei dieser Einzelkameras als Stereokamera agieren. In vorteilhafter Weise ermöglichen die unterschiedlichen Kombinationsmöglichkeiten der Einzelkameras zu einer Stereokamera eine breite Variation der möglichen Brennweiten des temporären Gesamtsystems und somit eine optimale Analyse der Areas-of-Interest (Aol).

Hierbei liefert eine geeignete Auswertung, z.B. unter Verwendung der dem Fachmann wohlbekannten Census Transformation und Kombination der Bilder einer jeden Einzel-Kamera ein Stereobild, welches auch Tiefeninformationen über die sich im Betrachtungsbereich befindlichen Personen und Gegenständen beinhaltet. Die Auswertung kann hierbei auf der Kamera selbst oder aber auf einem der Kamera zugeordneten Computer erfolgen. Je nach Ausstattung der Rechnerkomponenten sind durch Optimierung der Parameter Auflösung/Mono-Bildrate/Stereo-Bildrate/Gewählter Algorithmus/erlaubtes Delay (Echtzeitfähigkeit) unterschiedliche Performanceansprüche an das System möglich, welche eine optimale und effiziente Anpassung des Systems im Feld an die jeweiligen Umstände erlaubt.

Die gewonnen Tiefeninformationen können in einem nächsten Schritt weiter verwendet werden, um Personen im Bild zu isolieren, somit deren Präsenz zu detektieren und schließlich eine Positionsbestimmung durchführen zu können. Hierbei sind dem Fachmann aus der Literatur zahlreiche unterschiedliche Verfahren bekannt, wie etwa Verfahren, welche auf der Analyse und Extraktion von Texturen in einem Bild basieren, Verfahren, welche auf der Analyse und Interpolation von Bewegung über mehrere aufeinanderfolgende Bilder basieren, sowie modellbasierte Ansätze, welche im Kontext von Personenerkennung entweder monolytisch implementiert sein können, also den menschlichen Körper als Ganzes analysieren, oder teilebasiert, also einzelne Teile des menschlichen Körpers erkennen und diese gemäß geeigneter Hypothesen zu einem ganzen Körper zusammensetzen. Letzterer Ansatz eignet sich insbesondere für den Fall, dass eine partielle Verdeckung einer Person im Bild stattfindet.

Gemäß einer Ausgestaltung der Erfindung werden in einem nächsten Schritt im Rahmen einer Trainingsphase typische Teilkonturen der zu untersuchenden Objekte aus Bildsequenzen abgeleitet und in einer Datenbank gespeichert. Hierfür können zuvor die Texturen im zu untersuchenden Bild z.B. mit anisotropen Filtern hervorgehoben werden, welche typischerweise in 3D-Analysemethoden eingesetzt werden und in der Literatur wohlbekannt sind. Der Vorteil der Methode der Texturerkennung ist, dass in Abhängigkeit vom Anwendungsbereich unterschiedliche Templates hinterlegt werden können, welche die Resultate optimieren; beispielsweise werden für ein Zugangskontrollsystem eines Skigebietes Templates enthaltend einen Helm hinterlegt. Somit existiert ein optimales Set an Detektionsparametern für jeden Anwendungsfall. Da die erzeugten Texturen einfach sind, können diese auch leicht in Echtzeit Transformationen unterzogen werden, wie etwa Stauchungen, Rotationen oder Streckungen. Somit können diese Texturen im Bild auch dann wiedergefunden werden, wenn die Person sich dreht oder die Distanz zu Personen sich ändert.

In einem nächsten Schritt kann ein weiterer Satz an Informationen aus dem Bild erzeugt werden, welche durch die sogenannte "näherungsweise Textur-Kontext" Analyse gewonnen werden kann. Hierbei werden kleine, wenig komplexe 2D-Strukturen aus den Bildern extrahiert. Typische, bei menschlichen Körpern vorkommende Strukturen dieser Art werden danach in ein Codebook abgelegt. Hierbei können auch Informationen zur gegenseitigen lokalen Korrelation im Bild zwischen den einzelnen Strukturen hinterlegt werden. Mit geeigneten statistischen Methoden werden anschließend diese Einzelstrukturen auf Ihre Lage gegeneinander geprüft. Ergibt diese Prüfung, dass es auf Grund der Lage der Strukturen zueinander und der individuellen Eigenschaften der Strukturen im Einzelnen plausibel ist, dass eine Menge an diesen Strukturen eine menschliche Person beschreiben, so wird diese Submenge als solche gekennzeichnet und der entsprechende Bildbereich erhält eine (virtuelle) Boundingbox zur Indikation der Anwesenheit einer menschlichen Person.

In einem weiteren Schritt können obige Ansätze kombiniert werden, indem z.B. ein maximum a posteriori Schätzverfahren angewandt wird, welches dem Fachmann in diesem Bereich wohlbekannt ist. Das Ergebnis liefert einen robusten Schätzer für die individuelle Position und auch Lage (z.B. Körperhaltung, Körperdrehung, Kopfdrehen, Armausrichtung) der Personen im Bild.

Diese Information kann anschließend unter Kenntnis der Umgebung und der Lage der Kamera in absolute x-y-(z)-Koordinaten in der realen Umgebung umgewandelt werden, welche in weiterer Folge als Basis für die Lokalisierung, Zählung und Verfolgung (Tracking) von Personen genutzt werden können.

Mittels 3D- oder Trinocularkameras kann demnach eine Personenerfassung- und Erkennung durchgeführt werden.

Demnach werden die in einem Skigebiet, bei einer Messe oder in einem Freizeitpark anwesenden Personen in Echtzeit oder in -beispielsweise vorgegebenen - zeitlichen Abständen räumlich erfasst, so dass in einem Server eine People-Map erstellt wird, welche der räumlichen Verteilung der Personen entspricht. Durch die räumliche Erfassung der Personen als Funktion der Zeit wird ein Bewegungsprofil der Personen erstellt. Anhand der von den mobilen elektronischen Geräten gesendeten eindeutigen ID und/oder anhand der Daten der 3D-Kameras oder Trinocularkameras können die erfassten Personen getrackt werden.

Durch die Auswertung der People-Map ist es möglich, in dem Skigebiet, im Messegelände oder im Freizeitpark, die Entstehung von Warteschlangen oder größeren Ansammlungen vorherzusehen und zu vermeiden. Dies erfolgt durch eine Analyse der aktuellen und falls vorhanden von bekannten älteren Bewegungsprofilen von Einzelpersonen sowie der Gesamtheit der Personen anhand der People-Map. In Abhängigkeit der aktuellen Position der anwesenden Personen und der aktuellen Auslastung der Einrichtungen, können alle oder einzelne Personen von einem Server generierte Informationen erhalten, welche eine Redirektion dieser Personen bewirken können. Die Informationen sind vorzugsweise personenbezogen.

Ein Bewegungsprofil kann die Durchschnittsbewegungsgeschwindigkeit und die Bewegungsrichtung an Orten, welche eine Entscheidung über die Bewegungsrichtung erfordern, sowohl auf Einzelpersonen als auch auf Personengruppen bezogen, enthalten. Des Weiteren können die Bewegungsprofile die Aufenthaltszeiten von einzelnen Personen oder Gruppen an bestimmten Stellen, insbesondere vor Werbeplakaten, Hinweisschildern und dergl. enthalten. Des Weiteren können einer Person personenspezifische Informationen zugeordnet werden, beispielsweise Informationen über Altersgruppe oder Geschlecht, welche durch die Auswertung der Bilder von 3D-Kameras oder Trinocularkameras gewonnen werden können. Die Bewegungsprofile und falls vorhanden die personenspezifischen Informationen zu einer Person werden im Server einer erfassten Person und somit einem eindeutigen Besucherprofil zugeordnet. Jedem Besucherprofil ist eine eindeutige Besucher-ID zugeordnet.

Für den Fall, dass die Erfassung der Personen mittels mobiler elektronischer Geräte, z.B. Mobiltelefone, welche die Personen bei sich tragen, über einen Standard zur Kommunikation in Funknetzwerken erfolgt, wobei die Geräte in regelmäßigen Abständen eine eindeutige ID senden, können an den Eingängen 3D-Kameras oder Trinocularkameras vorgesehen sein, deren Bilder hinsichtlich personenspezifischer Informationen ausgewertet, mit der ID des jeweiligen mobilen elektronischen Gerätes verknüpft und einem Besucherprofil zugeordnet werden.

Die Bewegungsprofile und falls vorhanden personenspezifische Informationen werden ausgewertet, um das zukünftige Bewegungsprofil von Personen und Gruppen für ein bestimmtes vorgegebenes Zeitintervall vorherzusagen. Wenn sich beispielsweise eine Person lange vor einem Werbeplakat aufgehalten hat, wird diese Person mit einer hohen Wahrscheinlichkeit die beworbene Veranstaltung innerhalb der nächsten 30 Minuten besuchen. Wenn jedoch diese Person zuvor einen Essensstand besucht hat, wird sie die Veranstaltung mit einer hohen Wahrscheinlichkeit innerhalb von 15 Minuten besuchen, da weitere Essensstände auf dem Weg dorthin ignoriert werden.

Die Bewegungsprofile für Personen und/oder Personengruppen können während des aktuellen Besuchs der Personen oder der Gruppe gewonnen werden, wobei die Qualität der Daten mit der Zeit innerhalb des Besuchstages verbessert wird. Ferner können typische Verhaltensweisen sämtlicher Besucher innerhalb eines Tages evaluiert werden, um die Bewegungsprofile in Abhängigkeit von externen Faktoren, wie beispielsweise vom Wetter, zu erfassen.

Gemäß der Erfindung können in Abhängigkeit der Ergebnisse der Auswertung der Bewegungsprofile Maßnahmen ergriffen werden, um die Bildung von Warteschlangen zu vermeiden. Beispielsweise kann eine weitere Kasse geöffnet werden oder es können Personen durch Mitteilungen enthaltend geeignete Werbung oder Informationen umgeleitet werden, um die Bildung von Warteschlangen vor bestimmten Orten zu vermeiden und die Kapazität weiterer Einrichtungen auszunutzen.

Im Rahmen einer weiteren Ausgestaltung der Erfindung können Besucher proaktiv über Angebote informiert werden, wobei dies vorzugsweise mittels der Übermittlung von Push-Nachrichten auf die mobilen elektronischen Geräte mittels des verwendeten Funkstandards erfolgt.

Wenn einer erfassten Person anhand der vom mobilen elektronischen Gerät gesendeten ID oder anhand der Daten der 3D-Kameras oder Trinocularkameras ein bereits vorhandenes Besucherprofil zugeordnet werden kann, d.h. wenn es sich um einen wiederkehrenden Besucher handelt, wird im Server geprüft, ob Bewegungsprofile für dieses Besucherprofil aus der langfristigen Vergangenheit existieren, wobei, wenn dies der Fall ist, geprüft wird, ob ein Bewegungsprofil aus der kurzfristigen Vergangenheit, d.h. aus dem aktuellen Tag existiert. Wenn ein Bewegungsprofil aus der kurzfristigen Vergangenheit existiert wird geprüft, ob ein auswertbarer Kontext aus dem aktuellen Besuch vorhanden ist, was z.B. der Fall ist, wenn der Besucher mit Kindern da ist. Ist dies nicht der Fall, so werden die Bewegungsprofile aus der langfristigen und der kurzfristigen Vergangenheit kombiniert, um eine Vorhersage für die kurzfristige Zukunft, d.h. für ein bestimmtes vorgegebenes Zeitintervall in der Zukunft zu treffen. Für den Fall, dass ein auswertbarer Kontext existiert, wird dieser zusammen mit den Bewegungsprofilen aus der kurzfristigen und langfristigen Vergangenheit kombiniert, um die Vorhersage zu verbessern.

Wenn kein Bewegungsprofil aus der kurzfristigen Vergangenheit existiert und ein auswertbarer Kontext aus dem aktuellen Besuch existiert, werden die Bewegungsprofile aus der langfristigen Vergangenheit mit dem auswertbaren Kontext kombiniert, um die Vorhersage zu treffen. Sollte kein auswertbarer Kontext existieren, so werden die Bewegungsprofile aus der langfristigen Vergangenheit herangezogen, um die Vorhersage zu treffen.

Für den Fall, dass kein Bewegungsprofil aus der langfristigen Vergangenheit existiert und ein Bewegungsprofil aus der kurzfristigen Vergangenheit und/oder ein auswertbarer Kontext existieren, werden die vorhandenen Informationen zu einer Vorhersage der kurzfristigen Zukunft herangezogen. Existiert kein Bewegungsprofil aus der langfristigen und aus der kurzfristigen Vergangenheit, so werden die aktuellen Positionsinformationen zum Update der Bewegungsprofilinformation gespeichert.

Die gespeicherten Positionsinformationen werden zu einem Bewegungsprofil zusammengeführt, wobei aus den Vorhersageergebnissen Aktionen abgeleitet werden, um den Besucher derart zu führen, dass Staus und Warteschlangen vermieden werden, die Kapazität der Einrichtungen ausgenutzt wird und den Bedürfnissen des Besuchers optimal entsprochen wird.

Wenn der erfassten Person in der People-Map kein Besucherprofil zugeordnet werden kann, wird ein neues Besucherprofil angelegt und dieser Person zugeordnet. Dieses Besucherprofil wird mit der ID des mobilen elektronischen Gerätes, welches die Person bei sich trägt und/oder mit den Bildern von 3D-Kameras oder Trinocularkameras verknüpft.

Die Vorgehensweise entspricht der beschriebenen Vorgehensweise für den Fall, dass ein Besucherprofil bereits in der Datenbank existiert mit dem Unterschied, dass keine Bewegungsprofile aus der Vergangenheit existieren.

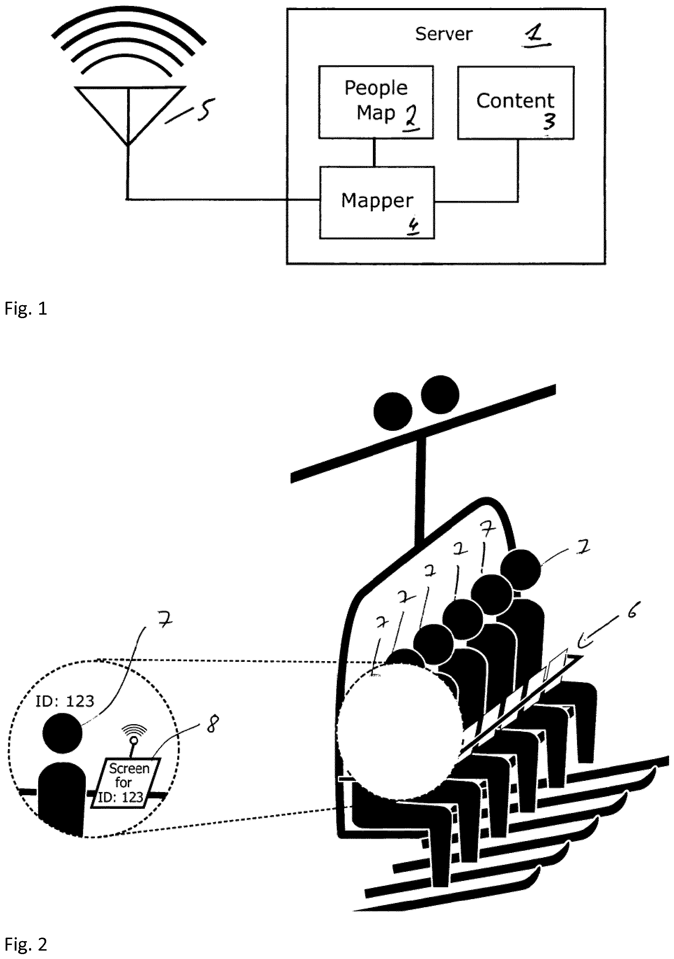

Anhand Figur 1 wird eine Ausgestaltung des Verfahrens beispielhaft erläutert. In Figur 1 ist ein Server 1 schematisch dargestellt. Gemäß der Erfindung wird im Server 1 eine People-Map 2 erstellt, welche der räumlichen Verteilung der Personen entspricht, wobei durch die räumliche Erfassung der Personen als Funktion der Zeit im Server 1 ein Bewegungsprofil der Personen erstellt wird. Ferner ist im Server eine Informations(Content)-Datenbank 3 vorgesehen, enthaltend die Besucherprofile, wobei mittels eines Mapping-Moduls (Mapper 4) in Abhängigkeit der aktuellen Position der anwesenden Personen, der Besucherprofile und der aktuellen Auslastung der Einrichtungen alle oder einzelne Personen generierte Informationen enthalten, die über eine geeignete Kommunikationsschnittstelle 5 versendet werden.

Die Informationen bzw. die Maßnahmen zum Führen der Personen können Push-Nachrichten oder SMS-Mitteilungen auf ein mobiles elektronisches Gerät der jeweiligen Person und/oder die Ansteuerung von dynamischen kontextsensitiven Werbetafeln in Abhängigkeit der aktuellen Position bestimmter Personen oder Gruppen sein. Im Rahmen einer Weiterbildung können die kontextsensitiven Werbetafeln einen Touch-Screen aufweisen, wobei die vor der Werbetafel stehende Person oder Personengruppe mittels akustischer und/oder optischer Signale, beispielsweise mittels blinkender Punkte auf den Touch-Screen zum Klicken auf spezifische Bereiche des Touchscreens animiert wird, um dort in der Folge Nachrichten zu erhalten, deren Inhalt das Verhalten der Person oder der Gruppe auf eine gewünschte Art und Weise beeinflussen kann.

Für den Fall, dass die Personen gemäß der Erfindung Push-Nachrichten erhalten, können diese mittels des zur Erfassung der Personen verwendeten Funkstandards oder über WLAN, vorzugsweise in Kombination mit einer spezifischen App gesendet werden. Die konkrete inhaltliche Anzeige der Nachrichten kann in Textform, auf Basis kontextbasierter geografischer Karten oder in Form einer konkreten Navigationsimplementierung mittels der spezifischen App erfolgen.

Ferner können die Push-Nachrichten, umfassend Informationen, Werbeeinblendungen, Navigationshinweise auf VR/AR-Brillen angezeigt werden.

Im Rahmen einer Weiterbildung der Erfindung können die Informationen, welche eine Redirektion dieser Personen bewirken können, mit einer im portablen elektronischen Gerät implementierten Geocaching Anwendung kombiniert werden, wobei die Personen Bonuspunkte erhalten, wenn sie Attraktionen in einer dynamisch definierbaren Abfolge ansteuern.

Die Redirektion der Benutzer kann alternativ oder zusätzlich auch optisch mittels einer mehrfarbigen in den Boden eingelassenen LED-Anordnung innerhalb eines begrenzten Areals oder mittels Lichtprojektionen auf den Boden, vorzugsweise mittels Laser-Projektoren erfolgen, wobei in Abhängigkeit der Position einzelner Personen oder Personengruppen einzelne Personen oder Gruppen mittels einer Ansteuerung der LED-Anordnung oder der Laser-Projektoren zu Einrichtungen gelotst werden können. Beispielsweise können durch die aktiven LEDs oder die Projektionen Pfeile gebildet werden, durch die eine bestimmte Richtung angezeigt wird.

Ferner kann jeder erfassten Person eine spezielle Farbe zugeordnet werden, wobei dies z.B. mittels eines farbigem Armbandes, welches die Personen am Eingang erhalten, durch Blinken von Status-LEDs in einer bestimmten Farbe am mobilen elektronischen Gerät erfolgen kann. Hierbei kann eine Person oder eine Gruppe durch farbige Signalgebung in dessen Umgebung in gewünschter Weise geleitet werden.

Alternativ oder zusätzlich können auch Mikrodrohnen eingesetzt werden, welche ausgewählten Personen oder Personengruppen fix oder dynamisch zugeordnet werden. Die Mikrodrohnen begleiten die Personen oder Gruppen entweder den ganzen Tag oder aber können Personen und Personengruppen bei Bedarf durch Verknüpfung der ID und Position der Personen oder Personengruppen mit der Position einer Mikrodrohne dynamisch zugeordnet werden. Die Mikrodrohnen können hierbei mit akustischen und/oder visuellen Signalisierungselementen ausgestattet sein; ferner kann in Kombination mit VR/AR-Brillen die Zuordnung einer Person oder Personengruppe auch im Display der VR/AR-Brille eingeblendet werden, wobei bei bestehender Zuordnung eine Audiowiedergabe mittels der VR/AR-Brille bei Betrachtung der Mikrodrohne erfolgen kann.

Im Rahmen einer weiteren Ausgestaltung, die insbesondere für Freizeitparks geeignet ist, können mittels als AR/VR-Brillen (Augmented Reality/Virtual Reality-Brillen) ausgeführten portablen Vorrichtungen, die jeweils einer Person zugeordnet sind, der jeweiligen Person virtuelle Schilder, Hinweise und/oder virtuelle Helfer angezeigt werden. Ferner kann auf den AR/VR-Brillen eine Geocaching App ausgeführt werden, mittels der die jeweilige Person Bonuspunkte bei einem Kundenbindungsprogramm erhalten kann, wenn sie Einrichtungen in einer dynamisch definierbaren Abfolge ansteuert.

Für den Fall eines Skigebietes umfassend Sesselbahnen wird gemäß einer Ausgestaltung der Erfindung vorgeschlagen, für jeden Sitz in einem Sessel eine Anzeigevorrichtung vorzusehen, über die der jeweiligen Person während der Fahrt Mitteilungen angezeigt werden, welche der Redirektion dieser Person dienen können. Wenn beispielsweise zum aktuellen Zeitpunkt in einem Restaurant des Skigebietes noch Plätze frei sind, und die Person noch kein Restaurant besucht hat, können der Person Informationen über dieses Restaurant und optional Sonderangebote angezeigt werden, welche auch den Erhalt von Bonuspunkten beim Besuch des Restaurants zum Gegenstand haben können, wenn die Person das Restaurant innerhalb einer vorgegebenen Zeit besucht. Ferner können der Person Alternativen bezüglich Skipisten angezeigt werden, die zum aktuellen Zeitpunkt ohne Warteschlangen an den Liften besucht werden können. Der Inhalt der Mitteilungen erfolgt vorzugsweise in Abhängigkeit des Bewegungsprofils der jeweiligen Person. Wenn die Person anhand des Bewegungsprofils überwiegend rot qualifizierte Pisten fährt, werden der Person entsprechende Alternativen angezeigt.

Diese Ausgestaltung wird anhand der beigefügten Figur 2 veranschaulicht. In Figur 2 ist ein Sessel 6 einer Sesselbahn dargestellt, welcher für sechs Personen 7 Platz bietet, wobei für jeden Sitz eine Anzeigevorrichtung 8 vorgesehen ist. Anhand der People-Map ist die Sitzposition der Personen 7 bekannt, so dass allen oder einzelnen Personen 7 über die jeweilige Anzeigevorrichtung 8 jeweils eine Mitteilung angezeigt wird, die vom jeweiligen Besucherprofil abhängt. Bei dem gezeigten Beispiel enthält Person mit der Besucher-ID 123 eine für sie bestimmte Mitteilung.

Im Rahmen einer Weiterbildung der Erfindung kann für jeden Sitz in einem Sessellift ein Beduftungsgerät vorgesehen sein, welches, wenn eine Anzeigevorrichtung vorgesehen ist, auch in die Anzeigevorrichtung integriert sein kann. Mittels des Beduftungsgerätes können Düfte versprüht werden, welche der Redirektion dieser Person dienen können und für den Fall, dass eine Anzeigevorrichtung vorgesehen ist, den über die Anzeigevorrichtung angezeigten Mitteilungen entsprechen; beispielsweise kann während der Anzeige freier Plätze in einem Restaurant ein entsprechender Duft versprüht werden; wird eine Wellness-Einrichtung angezeigt, mit dem Ziel, eine Redirektion der Person zu dieser Einrichtung zu bewirken, so kann ein Duft ätherischer Öle versprüht werden.

Für den Fall einer Gondelbahn kann pro Gondel eine Anzeigevorrichtung und/oder ein Beduftungsgerät vorgesehen sein, wobei in diesem Fall die Mitteilungen, welche der Redirektion dieser Person dienen können an alle Passagiere gerichtet sind.

Gemäß der Erfindung kann für den Fall eines Skigebietes umfassend Lifte und Zugangskontrollvorrichtungen zu den Liften an jeder Zugangskontrollvorrichtung zu den Liften des Skigebietes eine Anzeigevorrichtung vorgesehen sein, wobei mittels der Anzeigevorrichtung der Person, die vor der Anzeigevorrichtung steht, während der Fahrt Mitteilungen angezeigt werden, die der Redirektion dieser Person dienen können; der Inhalt der Mitteilungen erfolgt vorzugsweise in Abhängigkeit des Bewegungsprofils der jeweiligen Person.

## Patentansprüche

1. Verfahren zur Ausnutzung der Kapazität von Einrichtungen in einem Skigebiet, einer Messe, einem Freizeitpark oder in einem Stadion, **dadurch gekennzeichnet, dass** die aktuelle Position und das Bewegungsprofil sämtlicher im Bereich des Skigebiets, der Messe, des Freizeitparks oder des Stadions anwesenden Personen erfasst werden, wobei auf einem Server eine People-Map erstellt wird, welche der räumlichen Verteilung der Personen entspricht, wobei durch die räumliche Verteilung der Personen als Funktion der Zeit ein Bewegungsprofil der Personen erstellt wird, wobei in Abhängigkeit der aktuellen Position der anwesenden Personen, des Bewegungsprofils der anwesenden Personen und der aktuellen Auslastung der Einrichtungen, alle oder einzelne Personen vom Server generierte Informationen erhalten, welche eine Redirektion dieser Personen bewirken können, wodurch die Ausnutzung der Kapazität der Einrichtungen optimiert werden kann.

2. Verfahren nach Anspruch 1, **dadurch gekennzeichnet, dass** die Personen dadurch erfasst werden, dass mobile elektronische Geräte, welche die Personen bei sich tragen, über einen Standard zur Kommunikation in Funknetzwerken im Rahmen eines "Broadcasting" in regelmäßigen Abständen eine eindeutige ID senden, welche von mit einem Server verbundenen Sende-Empfangseinheiten in Reichweite des mobilen elektronischen Gerätes empfangen wird, wobei die Lokalisierung der Personen dadurch erfolgt, dass die mobilen elektronischen Geräte die mittels eines in die mobilen elektronischen Geräte integrierten GPS-Moduls gewonnenen aktuellen GPS-Daten zusammen mit der eindeutigen ID über den verwendeten Funkstandard an die sich in Reichweite befindenden Sende-Empfangseinheiten senden, dass die Personen anhand einer Personenerkennung mittels 3D-Kameras oder Trinocularkameras lokalisiert werden oder dass anhand einer Referenzsignalstärke für den verwendeten Funkstandard und die als Information im Signal des mobilen elektronischen Geräts enthalten ist oder in Abhängigkeit vom mobilen elektronischen Gerät, z.B. vom Typ des Mobiltelefons, in einer im Computer gespeicherten Tabelle abgelegt ist, der Abstand zwischen dem mobilen elektronischen Gerät und den sich in Reichweite befindenden Sende-Empfangseinheiten für den jeweiligen Funkstandard ermittelt wird, wobei anschließend eine Trilateration bzw. bei mehr als drei Sende-Empfangseinheiten eine Multilateration durchgeführt wird.

3. Verfahren nach Anspruch 2, **dadurch gekennzeichnet, dass** als Standard zur Kommunikation in Funknetzwerken ein Bluetooth Low Energy-Standard (BLE), ein WLAN-Standard oder ein UWB-Standard verwendet wird, wobei die eindeutige ID der IMEI - Nummer des mobilen elektronischen Gerätes entspricht.

4. Verfahren nach Anspruch 2 oder 3, **dadurch gekennzeichnet, dass** als mobiles elektronisches Gerät, mittels dessen die aktuelle Position sämtlicher im Bereich des Skigebiets, der Messe oder des Freizeitparks anwesenden Personen erfasst wird, eine portable Vorrichtung verwendet wird, die jeder Person beim Eintreten ausgehändigt wird, wenn diese nicht über diese Vorrichtung verfügen, welche ein Bauteil, welches die Kommunikation mit Sende-Empfangseinheiten des Skigebiets, der Messe, des Freizeitparks oder des Stadions mittels eines Funkstandards ermöglicht und optional eine Anzeigevorrichtung, ein Bauteil zur Ausgabe von akustischen Signalen und/oder ein GPS-Modul umfasst, wobei die portable Vorrichtung jeweils einer Person zugeordnet ist.

5. Verfahren nach Anspruch 4, **dadurch gekennzeichnet, dass** für den Fall eines Skigebiets die Vorrichtung eine Anzeigevorrichtung, ein Bauteil zur Ausgabe von akustischen Signalen und ein GPS-Modul aufweist und derart ausgeführt ist, dass sie an einem der Skier oder an einem Snowboard derart befestigbar ist, dass die Anzeigevorrichtung beim Skifahren durch den Benutzer sichtbar ist, wobei durch die Anzeigevorrichtung, wenn keine vom Server generierte Information angezeigt wird, die Geschwindigkeit, die Höhe und die gefahrenen Höhenmeter angezeigt werden.

6. Verfahren nach Anspruch 2, 3, 4 oder 5, **dadurch gekennzeichnet, dass** die vom Server generierten Informationen, welche eine Redirektion der Personen bewirken können, als Push-Nachrichten mittels des zur Erfassung der Personen verwendeten Funkstandards oder mittels WLAN oder als SMS-Mitteilungen auf das mobile elektronische Gerät der Personen übermittelt werden.

7. Verfahren nach einem der vorangehenden Ansprüche 2-6, **dadurch gekennzeichnet, dass** die vom Server generierten Informationen, welche eine Redirektion der Personen bewirken können, mittels der Ansteuerung von dynamischen kontextsensitiven Werbetafeln in Abhängigkeit der aktuellen Position bestimmter Personen oder Gruppen an bestimmte Personen oder Gruppen übermittelt werden.

8. Verfahren nach einem der vorangehenden Ansprüche 2-7, **dadurch gekennzeichnet, dass** die vom Server generierten Informationen, welche eine Redirektion der Personen bewirken können, optisch mittels einer mehrfarbigen in den Boden eingelassenen LED-Anordnung innerhalb eines begrenzten Areals oder mittels Lichtprojektoren übermittelt werden, wobei in Abhängigkeit der Position einzelner Personen oder Personengruppen einzelne Personen oder Gruppen mittels einer Ansteuerung der LED-Anordnung oder der Lichtprojektoren zu Einrichtungen gelotst werden können.

9. Verfahren nach einem der vorangehenden Ansprüche 2-8, **dadurch gekennzeichnet, dass** die vom Server generierten Informationen, welche eine Redirektion der Personen bewirken können, mittels Mikrodrohnen übermittelt werden, die ausgewählten Personen oder Personengruppen fix oder dynamisch zugeordnet werden, wobei die Mikrodrohnen die Personen oder Gruppen entweder den ganzen Tag begleiten oder Personen und Gruppen bei Bedarf durch Verknüpfung der ID und Position der Personen mit der Position einer Mikrodrohne dynamisch zugeordnet werden, wobei die Mikrodrohnen mit akustischen und/oder visuellen Signalisierungselementen ausgestattet sind.

10. Verfahren nach einem der vorangehenden Ansprüche 2-9, **dadurch gekennzeichnet, dass** für den Fall eines Skigebietes umfassend Sesselbahnen, für jeden Sitz in einem Sessel eine Anzeigevorrichtung vorgesehen ist, über die der jeweiligen Person während der Fahrt Mitteilungen angezeigt werden, welche der Redirektion dieser Person dienen können.

11. Verfahren nach einem der vorangehenden Ansprüche 2-10, **dadurch gekennzeichnet, dass** für den Fall eines Skigebietes umfassend Sesselbahnen, für jeden Sitz in einem Sessellift ein Beduftungsgerät vorgesehen ist, mittels dessen Düfte versprühbar sind, welche der Redirektion dieser Person dienen können, wobei, wenn für jeden Sitz eine Anzeigevorrichtung vorgesehen ist, die Düfte den über die Anzeigevorrichtung angezeigten Mitteilungen entsprechen.

12. Verfahren nach einem der vorangehenden Ansprüche 2-11, **dadurch gekennzeichnet, dass** für den Fall eines Skigebietes umfassend Lifte und Zugangskontrollvorrichtungen zu den Liften, an jeder Zugangskontrollvorrichtung zu den Liften des Skigebietes eine Anzeigevorrichtung vorgesehen ist, wobei mittels der Anzeigevorrichtung der Person, die vor der Anzeigevorrichtung steht, Mitteilungen angezeigt werden, die der Redirektion dieser Person dienen können.

13. Verfahren nach einem der vorangehenden Ansprüche 2-12, **dadurch gekennzeichnet, dass** der Inhalt der Mitteilungen, welche eine Redirektion der Personen bewirken können, in Abhängigkeit der aktuellen Position der Personen, der aktuellen Auslastung der Einrichtungen und durch eine Analyse der aktuellen und falls vorhanden von bekannten älteren Bewegungsprofilen von Einzelpersonen sowie der Gesamtheit der Personen anhand der People-Map bestimmt wird, wobei ein Bewegungsprofil die Durchschnittsbewegungsgeschwindigkeit und die Bewegungsrichtung an Orten, welche eine Entscheidung über die Bewegungsrichtung erfordern, sowohl auf Einzelpersonen als auch auf Personengruppen bezogen, enthält und wobei ein Bewegungsprofil optional die Aufenthaltszeiten von einzelnen Personen oder Gruppen an bestimmten Stellen enthält, wobei Personen optional personenspezifische Informationen zugeordnet werden, welche durch die Auswertung der Bilder von 3D-Kameras oder Trinocularkameras gewonnen werden, wobei die Bewegungsprofile und falls vorhanden die personenspezifischen Informationen zu einer Person im Server der über den verwendeten Funkstandard gesendeten eindeutigen ID und somit einem eindeutigen Besucherprofil zugeordnet werden.

14. Verfahren nach Anspruch 13, **dadurch gekennzeichnet, dass** die Bewegungsprofile und falls vorhanden personenspezifische Informationen ausgewertet werden, um das zukünftige Bewegungsprofil von Personen und Gruppen für ein bestimmtes vorgegebenes Zeitintervall vorherzusagen, wobei die Bewegungsprofile für Personen und/oder Gruppen während des aktuellen Besuchs der Personen oder der Gruppe gewonnen werden, wobei die Qualität der Daten mit der Zeit innerhalb des Besuchstages verbessert wird, wobei in Abhängigkeit der Ergebnisse der Auswertung der Bewegungsprofile Maßnahmen ergriffen werden, um die Bildung von Warteschlangen zu vermeiden, wobei entsprechende Mitteilungen an alle oder einzelne Personen übermittelt werden.

15. Verfahren nach Anspruch 13 oder 14, **dadurch gekennzeichnet, dass** wenn einer erfassten Person anhand der vom mobilen elektronischen Gerät der Person gesendeten ID oder anhand der Daten von 3D-Kameras oder Trinocularkameras ein bereits vorhandenes Besucherprofil zugeordnet werden kann, im Server geprüft wird, ob Bewegungsprofile für dieses Besucherprofil aus der langfristigen Vergangenheit existieren, wobei, wenn dies der Fall ist, geprüft wird, ob ein Bewegungsprofil aus der kurzfristigen Vergangenheit, d.h. aus dem aktuellen Tag existiert, wobei, wenn ein Bewegungsprofil aus der kurzfristigen Vergangenheit existiert, geprüft wird, ob ein auswertbarer Kontext aus dem aktuellen Besuch vorhanden ist, wobei, wenn dies nicht der Fall ist, Bewegungsprofile aus der langfristigen und der kurzfristigen Vergangenheit kombiniert werden, um eine Vorhersage für die kurzfristige Zukunft, d.h. für ein bestimmtes vorgegebenes Zeitintervall in der Zukunft zu treffen und wobei, wenn ein auswertbarer Kontext aus dem aktuellen Besuch existiert, dieser zusammen mit den Bewegungsprofilen aus der kurzfristigen und langfristigen Vergangenheit kombiniert wird, um die Vorhersage zu verbessern, wobei, wenn kein Bewegungsprofil aus der kurzfristigen Vergangenheit und ein auswertbarer Kontext aus dem aktuellen Besuch existiert, die Bewegungsprofile aus der langfristigen Vergangenheit mit dem auswertbaren Kontext kombiniert werden, um die Vorhersage zu treffen, wobei wenn kein Bewegungsprofil aus der kurzfristigen Vergangenheit und kein auswertbarer Kontext aus dem aktuellen Besuch existieren, die Bewegungsprofile aus der langfristigen Vergangenheit herangezogen werden, um die Vorhersage zu treffen, wobei, wenn kein Bewegungsprofil aus der langfristigen Vergangenheit existiert und ein Bewegungsprofil aus der kurzfristigen Vergangenheit und/oder ein auswertbarer Kontext existieren, die vorhandenen Informationen zu einer Vorhersage der kurzfristigen Zukunft herangezogen werden und wobei, wenn kein Bewegungsprofil aus der langfristigen und aus der kurzfristigen Vergangenheit existiert, die aktuellen Positionsinformationen zum Update der Bewegungsprofilinformation gespeichert werden und wobei, wenn einer erfassten Person kein Besucherprofil zugeordnet werden kann, ein neues Besucherprofil angelegt und dieser Person zugeordnet wird, wobei dieses Besucherprofil mit der ID des mobilen elektronischen Gerätes, welches die Person bei sich trägt und/oder mit den Bildern von 3D-Kameras oder Trinocularkameras verknüpft wird.
